# EUROPÄISCHE PATENTSCHRIFT

(11) **EP 3 166 874 B1**
(45) Veröffentlichungstag und Bekanntmachung des Hinweises auf die Patenterteilung: **13.06.2018**
(21) Anmeldenummer: 15770783.7
(22) Anmeldetag: 11.07.2015
(51) Int. Cl.: B65F 1/06, A47K 11/02, B65B 67/12, B65B 9/02

(54) **VORRICHTUNG SOWIE UMHÜLLUNGSVERFAHREN ZUM PORTIONSWEISEN VERSCHLIESSEN VON EXKREMENTEN, VERBANDSMATERIALIEN, DAMENHYGIENEARTIKELN ODER DERGLEICHEN IN UMHÜLLUNGEN**
DEVICE AND WRAPPING METHOD FOR INDIVIDUALLY WRAPPING EXCREMENTS, WOUND DRESSING MATERIALS, FEMALE HYGIENE ARTICLES OR THE LIKE IN WRAPPERS
DISPOSITIF ET PROCÉDÉ D'EMBALLAGE SERVANT À ENFERMER, SOUS FORME DE PORTIONS, DES EXCRÉMENTS, MATÉRIAUX DE BANDAGE, ARTICLES D'HYGIÈNE FÉMININE OU SIMILAIRES DANS DES EMBALLAGES

(30) Priorität: 11.07.2014 DE 102014010264
(43) Veröffentlichungstag der Anmeldung: 17.05.2017
(73) Patentinhaber: Rotho Babydesign GmbH, 79733 Görwihl (DE)
(72) Erfinder: SCHMITT, Fritz, 6579 Rosport (LU)
(74) Vertreter: Westphal, Mussgnug & Partner Patentanwälte mbB
(86) Internationale Anmeldenummer: PCT/DE2015/000346
(87) Internationale Veröffentlichungsnummer: WO 2016/004916

(56) Entgegenhaltungen:
- EP-A1- 0 906 743
- EP-A1- 2 447 192
- EP-A2- 2 441 684
- GB-A- 2 502 448
- JP-A- 2005 145 562
- NL-C2- 1 019 558

## Beschreibung

Die vorliegende Erfindung betrifft eine Vorrichtung gemäß dem Oberbegriff des Anspruchs 1 sowie ein Umhüllungsverfahren zum portionsweisen Verschließen von Exkrementen, Verbandsmaterialien, Damenhygieneartikeln oder dergleichen in Umhüllungen. Eine gattungsgemäße Vorrichtung und ein entsprechendes Verfahren zur Umhüllung von Materialien sind aus der GB 2 502 448 A bekannt. Aus EP 1 324 919 B1 ist eine Vorrichtung zur getrennten Abdichtung mehrerer Objekte bekannt, die eine Aufnahme von kontaminiertem Umhüllungsgut durch ein rotierbares Ringmagazin hindurch gestattet.

Es ist Ziel der Erfindung eine verbesserte Lösung zur Umhüllung solcher Objekte, beispielsweise Exkremente, Verbandsmaterialien, Damenhygieneartikeln, Windeln oder dergleichen, zur Verfügung zu stellen. Verbesserungsbedarf wird insbesondere gesehen in Bezug auf Materialverbrauch, portionsweise dichter Verschlusstechnik, Handhabung.

Die Aufgabe wird erfindungsgemäß mittels einer Vorrichtung gemäß Anspruch 1 umfassend seine kennzeichnenden Merkmale sowie mittels eines Verfahrens gemäß Anspruch 11 gelöst, sowie durch deren Verwendung nach Anspruch 16. Vorteilhafte Ausführungsformen sind in den jeweiligen Unteransprüchen angegeben.

Eine gattungsgemäße Vorrichtung ist zum portionsweisen Verschließen von Exkrementen, Verbandsmaterialien, Damenhygieneartikeln oder dergleichen in Umhüllungen geeignet und weist auf: einen Behälter und/oder einen Deckelaufsatz für einen Behälter mit zumindest einer Befüllöffnung zum Aufnehmen zumindest eines zu verschließenden Objekts, nämlich eines Umhüllungsguts, ferner einen zumindest teilweise in dem Behälter (10) und/oder dem Deckelaufsatz aufgenommenen Bewegungskoordinator für einen Vortrieb des Objekts in zumindest einer Bewegungsrichtung. Die Vorrichtung hat zumindest ein Magazin zur Abgabe von zumindest zwei Umhüllungsmaterialien. Zumindest ein Oberflächenbereich des Bewegungskoordinators ist in Bewegungsrichtung auf das Magazin folgend angeordnet, so auch die Befüllöffnung. Der Bewegungskoordinator passiert zumindest mit dem Oberflächenbereich während der Bewegung den Bereich hinter dem Magazin, in dem die Befüllöffnung angeordnet ist. Das Umhüllungsverfahren arbeitet zum portionsweisen Verschließen von Exkrementen, Verbandsmaterialien, Damenhygieneartikeln oder dergleichen in Umhüllungen mittels einer erfindungsgemäßen Vorrichtung, mit einer Transportvorrichtung, die bevorzugt ein Teil des Bewegungskoordinators ist, für ein erstes Umhüllungsmaterial. Die Transportvorrichtung bildet gemeinsam mit dem ersten Umhüllungsmaterial eine Aufnahmevertiefung aus. Das Umhüllungsverfahren arbeitet weiterhin mit einem zweiten Umhüllungsmaterial, das bevorzugt in einer Verschlusseinrichtung bevorratet ist, welche die Aufnahmevertiefung zu verschließen bestimmt ist, und einem zum Verschließen bewegbaren Körper. Dabei sorgt eine einzige Bewegung des Körpers erstens für ein Entnehmen des ersten Umhüllungsmaterials aus dem ggf. ersten Magazin, bevorzugt zweitens für ein Verdichten des in die Aufnahmevertiefung eingelegten Umhüllungsguts und immer aber drittens für ein Verschließen der Umhüllung mittels des zweiten Umhüllungsmaterials aus dem selben Magazin oder einem zweiten Magazin. Dies geschieht, indem die Verschlusseinrichtung, bevorzug unter Zusammenwirkung mit dem Bewegungskoordinator, beide Umhüllungsmaterialien um die Aufnahmevertiefung herum in einer Dichtzone in Kontakt bringt.

Die Verwendung von zwei Magazinen gestattet eine sparsame Verwendung von kostengünstig mit unterschiedlichen Eigenschaften getrennt beziehbarem Umhüllungsmaterial, wodurch insgesamt das aufkommende Müllvolumen so gering wie möglich gehalten wird. Die Verwendung von zwei Umhüllungsmaterialien statt einem einzigen Umhüllungsmaterial ermöglicht es ferner, die Umhüllung während des Umhüllungsprozesses beeinflussen zu können, insbesondere ein Verdichten der Umhüllung. Das zum erfindungsgemäßen Umhüllen vorgeschlagene Verfahren sowie die unabhängig dazu vorgeschlagene Vorrichtung ermöglichen eine komfortable Handhabung mit beispielsweise nur einem einzigen Hub, wobei dieser Hub bei Aufteilung der Arbeitsschritte über den Umfang der Trommel bzw. des Körpers das erste Umhüllungsmaterial entnimmt und zur Aufnahme vorbereitet sowie gleichzeitig ein eingelegtes Gut als echte Portion umhüllt.

Nach einer bevorzugten Ausführungsform bestimmt alleine die Größe des Objekts die Größe der späteren Umhüllung. Es ist jedoch Eigenschaft vorteilhafter Ausführungsformen, dass zusätzlich mittels der erfindungsgemäßen Vorrichtung nach einer solchen bevorzugten Ausführungsform die bereits oben erwähnte Komprimierung des Objekts erreicht wird, bevor die Umhüllung schließt. Nach dem Verschließen ist die erfindungsgemäße Umhüllung besonderer Ausführungsformen über eine Gasdichtheit der Umhüllungsmaterialien selbst und deren Verbindung hinaus dazu in der Lage, die Komprimierung des Objekts zu fixieren oder quasi einzufrieren, in dem die Umhüllung Kräften des zur erneuten Ausdehnung neigenden Objekts und ggf. den zusätzlichen Kräften durch Raumforderungen austretender Gase, die eben nicht durch die Umhüllung herausgelassen werden, standhält.

Gemäß einer vorteilhaften Ausführungsform der Vorrichtung ist die Rotationsachse der Trommel zumindest annähernd horizontal oder vertikal ausgerichtet.

Die horizontale Ausrichtung der Trommel bewirkt eine Bewegbarkeit des zentralen Verfahrenskörpers mittels eines vertikalen Hubs, der beispielsweise mittels eines Fußpedals und der Kraft eines menschlichen Beines ausgeführt wird. Diese Kraftwirkungsrichtung entspricht besonders gut den Aufnahmemöglichkeiten beispielsweise bei Aufstellung der Vorrichtung auf einem ebenen Fußboden.

Gemäß einer weiteren vorteilhaften Ausführungsform der Vorrichtung sind die Magazine mit Umhüllungsrotationsachsen parallel zu der Rotationsachse der rotierbaren Trommel ausgerichtet angeordnet.

Sind sämtliche Achsausrichtungen zu einander parallel angepasst ergibt sich ein minimaler Umhüllungsmaterialverbrauch.

Gemäß einer weiteren vorteilhaften Ausführungsform der Vorrichtung ein Zwickel zwischen einer im Behälter befestigten oder ausgeformten Gegenlage und der Trommel ausgebildet. Die Ausführung eines solchen sich verjüngenden Bereichs im Transportweg der Umhüllung, insbesondere während des Verschließens, lässt die Bewegungskraft des zentralen Verfahrenskörpers das Verdichten bewirken und das Abdichten der Portion verbessern.

Gemäß einer weiteren vorteilhaften Ausführungsform der Vorrichtung ist der Befüllöffnung zumindest ein in der Trommel angeordneter Boden zugeordnet ist, der je nach Stellung der Trommel etwa flächenkongruent zur Befüllöffnung angeordnet arretierbar.

Somit ist die Handhabbarkeit verbessert und das Umhüllungsergebnis gesichert. Das Umhüllungsmaterial muss den Kräften beim Befüllen mit Umhüllungsgut nicht alleine entgegentreten. Es wird durch den Boden gestützt.

Besonders bevorzugt weist der Boden bei Betrachtung eines Radialschnitts in Rotationsrichtung gesehen einen kleineren Einlaufradius und einen größeren Auslaufradius auf.

Damit fördert auch der Boden das Verdichten während des Umhüllungstransports.
Gemäß einer weiteren vorteilhaften Ausführungsform der Vorrichtung weist die Trommel über ihren Umfang bzw. ihre Lauffläche verteilt mehrere von Dichtzonen umrandete Aufnahmevertiefungen auf, wobei die Dichtzonen Teilflächen einer gedachten Umfangsfläche der Trommel sind.

Solche Dichtzonen können, bevorzugt mit Noppungen in den Dichtflächen der Trommel, außer einer Steigerung der Dichthaltbarkeit auch den Umhüllungsmaterialtransport zumindest präzisieren.

Gemäß einer weiteren vorteilhaften Ausführungsform der Vorrichtung ist eine Andruckrolle des zweiten Magazins Gegenlage für das Verdichten des Umhüllungsguts und Dichtandruckfläche zugleich.
Als Vorteil werden Materialeinsparung und Leichtgängigkeit genannt.

Gemäß der vorliegenden Erfindung sind in einem zentralen Bereich der Trommel zumindest ein Auswerfer angeordnet und die Trommel axial mittig zumindest bereichsweise radial offen ausgestaltet. Der Auswerfer erreicht mithin im Umfangsbereich dem zweiten Magazin in Rotationsrichtung nachgeschaltet das umhüllte Material, wobei er dasselbe auswirft. Bevorzugt lenkt die Umhüllung den Auswerfer gegen die Kraft einer Feder aus. Diese Einrichtung verbessert die Betriebssicherheit.
Gemäß weiteren vorteilhaften Ausführungsformen der Vorrichtung weist der Behälter zumindest eines der folgenden Merkmale auf:
- gasdicht verschließbar,
- abschließbar, insbesondere mit einem Zahlenschloss,
- enthaltend einen Zähler und eine Schnittstelle zur Datenübertragung,
- aufweisend ein Display zur Anzeige der Füllmenge und / oder von Geruchsgefahr,
- aufweisend eine Kamera,
- aufweisend eine Fangschale für Feuchtigkeit, insbesondere mit einer Geruchssperrschicht, insbesondere Glycerin,
- doppelwandig mit Absorptionsmaterial zwischen den Wänden,
- aufweisend eine Beleuchtungseinrichtung und einen Energiespeicher, wobei der Energiespeicher beim Antreiben der Trommel und/oder Solarenergie aufladbar ist,
- aufweisend eine Atmosphäre im Inneren, insbesondere ein sterilisierendes Gas,
- aufweisend eine Fallklappe mit Spannlippe als Dichtung,
- aufweisend ein Fenster mit Vergrößerungsglas zur intensiven Beleuchtung des Behältervolumens.

Gemäß einem vorteilhaften Umhüllungsverfahren findet um die Aufnahmevertiefung vollständig herumgeführt in der Dichtzone ein Kaltverschweißen statt, um verdichtetes Umhüllungsgut hermetisch gegenüber der Umwelt abzuriegeln.
Gemäß einer weiteren vorteilhaften Ausführungsform des erfindungsgemäßen Umhüllungsverfahrens wird das Verdichten dem Umhüllungsverfahren nachgeschaltet zunächst durch einen Schrumpfprozess verstärkt, wobei selbiges Schrumpfen ausgelöst wird durch einen Kontakt zwischen Umhüllungsmaterial und Umhüllungsgut. Es stellt sich dabei erfindungsgemäß eine Gasdichtheit der Umhüllungsmaterialien erst nach dem Schrumpfprozess ein.
Gemäß einer weiteren vorteilhaften Ausführungsform des erfindungsgemäßen Umhüllungsverfahrens ist je zu verschließender Portion Umhüllungsgut ein einziger, antreibender Hub erforderlich, der auf die Transportvorrichtung wirkt.

Gemäß des erfindungsgemäßen Umhüllungsverfahrens treibt der Antrieb des Körpers eine aktive Vakuumierungseinrichtung, insbesondere eine Pumpe, mit an. Ein Anschluss zwischen der Umhüllung und der Vakuumierungseinrichtung bleibt dabei solange geöffnet, bis ein hinteres Ende der Umhüllung abgedichtet wird.

Es ist je nach Umhüllungsgut aus Aufbewahrungssicht und / oder aus Verwertungssicht oft vorteilhaft beispielsweise Oxidationen weitgehend zu vermeiden.

Erfindungsgemäße Vorrichtungen oder Umhüllungsverfahren werden bevorzugt mit Materialien ausgerüstet, die alle vollständig biologisch abbaubar sind.

Diesen Merkmalen sind folgende Vorteile zuzuordnen: keine Geruchsbelastung; einfache Funktion, insbesondere Einhandbedienung; Lagerfähigkeiten der umhüllten Umhüllungsgüter bis zu 3 Wochen; Verwendung von für die Umwelt unbedenklichen Materialien. Beispielsweise können derart umhüllte Windeln gemäß einer erfindungsgemäßen Vorrichtung unter Vermeidung von Verlusten zur Kompostierung oder Müllverbrennung vorbereitet werden.

Bevor nachfolgend Ausgestaltungen der Erfindung eingehender beschrieben werden, ist zunächst festzuhalten, dass die Erfindung nicht auf die beschriebenen Komponenten oder die beschriebenen Verfahrensschritte beschränkt ist. Weiterhin stellt auch die verwendete Terminologie keine Einschränkung dar, sondern hat lediglich beispielhaften Charakter. Soweit nachfolgend in der Beschreibung und den Ansprüchen der Singular verwendet wird ist dabei jeweils der Plural mitumfasst, soweit der Kontext dies nicht explizit ausschließt.

Obwohl die Erfindung in den Zeichnungen und der vorausgegangenen Darstellung im Detail beschrieben ist, sind die Darstellungen illustrativ und beispielhaft und nicht einschränkend zu verstehen. Insbesondere ist die Wahl der zeichnerisch dargestellten Proportionen der einzelnen Elemente nicht als erforderlich oder beschränkend auszulegen. Weiterhin ist die Erfindung insbesondere nicht auf die erläuterten Ausführungsbeispiele beschränkt. Weitere Varianten der Erfindung und ihre Ausführung ergeben sich für den Fachmann aus der vorangegangenen Offenbarung, den Figuren und den Schutzansprüchen.

In den Schutzansprüchen verwendete Begriffe wie "umfassen", "aufweisen", "beinhalten", "enthalten" und dergleichen schließen weitere Elemente oder Schritte nicht aus. Die Verwendung des unbestimmten Artikels schließt eine Mehrzahl nicht aus. Eine einzelne Einrichtung kann die Funktionen mehrerer in den Schutzansprüchen genannten Einheiten bzw. Einrichtungen ausführen.

Nachfolgend wird die Erfindung anhand eines in Figuren dargestellten Ausführungsbeispiels näher erläutert. Es zeigen
- Fig. 1: eine Seitenansicht entlang einer Radiallinie auf das Innenleben einer erfindungsgemäßen Vorrichtung gemäß einem Ausführungsbeispiel, nämlich auf eine Trommel mit darüber und darunter angeordneten Magazinrollen für Umhüllungsmaterial,
- Fig. 2a: eine unvollständige Darstellung eines Radialschnitts durch die erfindungsgemäße Vorrichtung gemäß dem in Fig. 1 eingetragenen Schnitt II-II nach Einlegen eines Umhüllungsguts,
- Fig. 2b: die Darstellung gemäß Fig. 2a während eines Verdichtens des Umhüllungsguts,
- Fig. 3a: eine unvollständige Darstellung eines weiteren Radialschnitts durch die erfindungsgemäße Vorrichtung gemäß dem in Fig. 1 eingetragenen Schnitt III-III nach dem Verdichten und kurz vor Kontakt mit einem Auswerfer,
- Fig. 3b: die Darstellung gem. Fig. 3a nach einem Auswerfen mit entspanntem Auswerfer,
- Fig. 4: eine Seitenansicht auf eine schematisch dargestellte Trommel gemäß dem bisher in den Figuren 1 bis 3 dargestellten Ausführungsbeispiel mit ebenso schematischer Darstellung einer Antriebsvariante,
- Fig. 5: eine perspektivische Außenansicht auf die erfindungsgemäße Vorrichtung gemäß dem Ausführungsbeispiel der bisherigen Figuren 1 bis 4 und
- Fig. 6: eine perspektivische Ansicht auf die in Figur 1 dargestellten Teile des Ausführungsbeispiels.

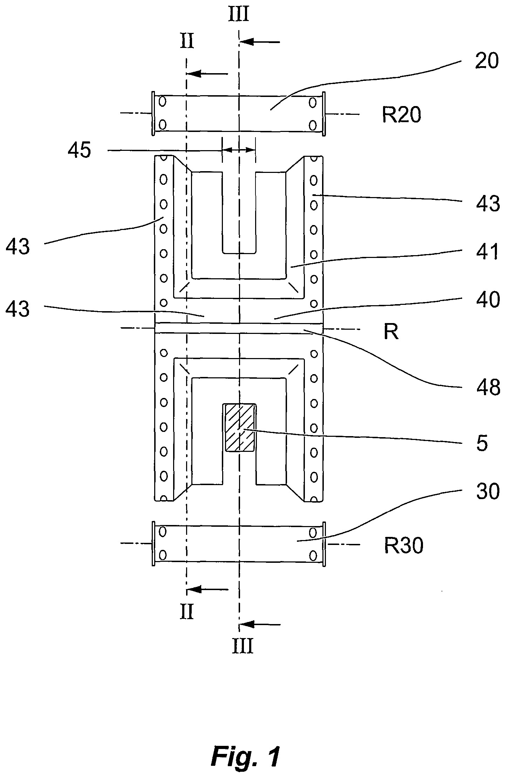

In den Figuren 1 bis 6 ist ein Ausführungsbeispiel einer erfindungsgemäßen Vorrichtung gemäß obiger Auflistung dargestellt, mit der erfindungsgemäß umhüllt werden kann. Sämtliche Figuren enthalten gleiche Bezugszeichen für gleiche Teile. Es sind jedoch zur Erhaltung der Übersichtlichkeit ggf. nicht in allen Figuren alle eintragbaren Bezugszeichen eingetragen.

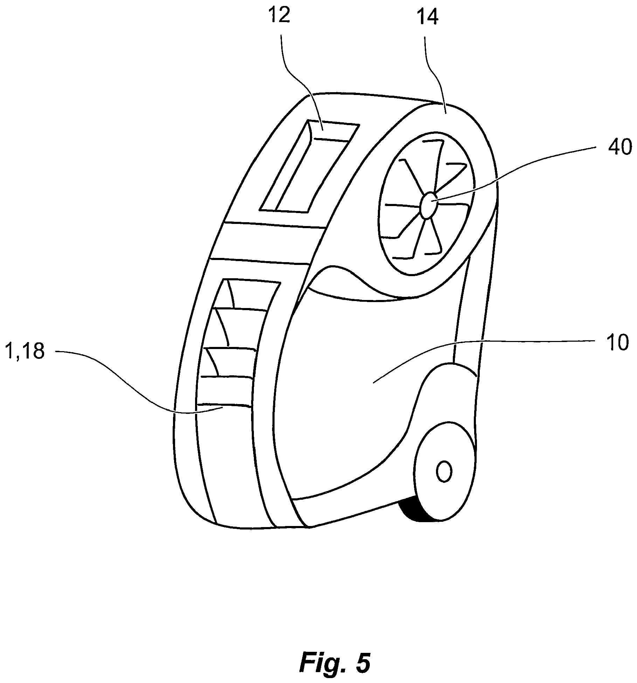

Eine Übersicht von außen auf die Vorrichtung wird in Fig. 5 gewährt. Schematisch dargestellt ist ein Behälter 10 zur Aufbewahrung von Umhüllungen geruchsdicht abgedichtet enthaltend Exkremente, Verbandsmaterialien, Damenhygieneartikel oder dergleichen. Auf dem Behälter sitzt ein Aufsatz 14, der wesentliche funktionelle Bauteile der Vorrichtung aufnimmt. Darunter ist ein zentraler Körper der Vorrichtung erkennbar, nämlich eine Trommel 40 in vereinfachter und technisch unpräziser Darstellung und Dimensionierung.

Die perspektivische Ansicht der Fig. 5 gestattet ferner einen Blick auf die Frontseite der Vorrichtung mit oben angedeuteter Befüllöffnung 12 zum Befüllen mit Umhüllungsgut und unten angedeutetem Fußpedal 18 zum Antrieb der Trommel 40.

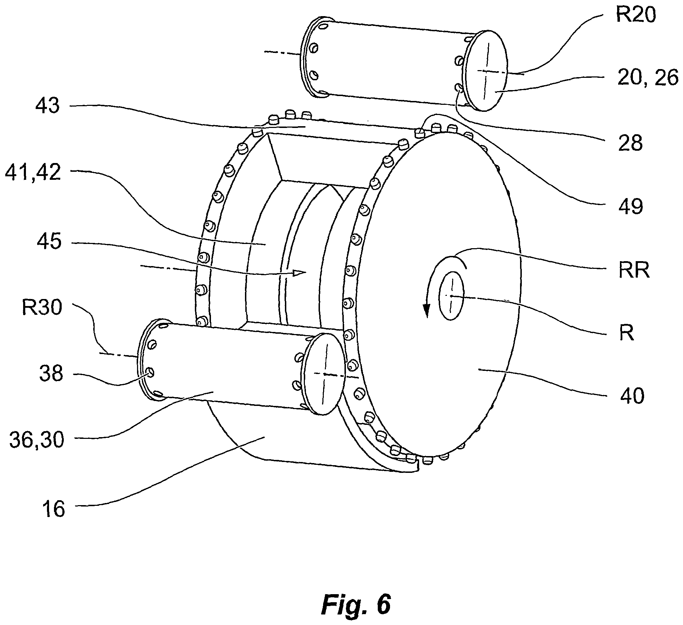

Die Figuren 1 und 6 gestatten einen Blick ins Innere des Aufsatzes 14. Technisch immer noch nur schematisch dargestellt sind die Trommel 40, die mittels des Fußpedals 18 um eine Rotationsachse R drehbar ist. Ferner sind um deren Umfang ober- und unterhalb der in Fig. 5 dargestellten Befüllöffnung 12 koaxial R20, R30 zur Rotationsachse R ein erstes 20 und ein zweites Magazin 30 mit nach Darstellungsweise einer Explosionsdarstellung radial entfernten Rollen 26, 36 vermerkt. Sie sind dazu bestimmt, auf der Trommel 40 abzurollen. Es kann zum Materialtransport und zu Abdichtungszwecken erforderlich sein, die Kontaktflächen zu topographieren, was mit Noppen 49 und Näpfen 28, 38 angedeutet ist.

In Umfangsrichtung RR der Trommel 40 weiter der durch den Antrieb der Trommel 40 vorgegebenen Rotationsrichtung RR folgend ist in Fig. 6 etwa auf die zweite Rolle 36 des zweiten Magazins 30 folgend eine sich an die Trommel 40 in Umfangsrichtung zunehmend anschmiegende Gegenlage 16 dargestellt. Sie bewirkt ein Verdichten des auch hier noch nicht dargestellten Umhüllungsguts. Die Magazine 20, 30, die Trommel 40 und die Gegenlage 16 sind zumindest zeitlich im Aufsatz 14 aufgenommen.

Im Rahmen der Erfindung ist auch eine Ausführungsform umfasst, welche eine Aufnahme direkt im Behälter hat, um auf einen Aufsatz zu verzichten. Ebenso umfasst die Erfindung Einsätze in bestehende Einrichtungen, welche die Vorrichtung aufnehmen, beispielsweise in Krankenhauseinrichtungen oder Flugzeugausstattungen.

Bei genauerem Blick durch die Befüllöffnung 12 der Fig. 5, der in Fig. 6 gestattet ist, erkennt der Betrachter eine Aufnahmevertiefung 41 für das hier noch nicht dargestellte Umhüllungsgut, mit einem Boden 42. Der Boden 42 ist axial R mittig in einem zentralen Bereich 45 unterbrochen, um einem in Fig. 1 schraffiert angedeuteten Auswerfer 5 einen radialen Durchtritt zu ermöglichen. Der Durchtritt erfolgt in Umfangsrichtung der Rotationsrichtung RR der Gegenlage 16 folgend nach unten (nicht in Fig. 6 dargestellt).

In Fig. 1 sind zwei der in Umfangsrichtung über die Trommel verteilten Aufnahmevertiefungen 41 erkennbar. Die Aufnahmevertiefungen können im Rahmen der Erfindung unterschiedlicher Größe sein, um beispielsweise verschiedene Windelgrößen zu berücksichtigen. Zwischen den Aufnahmefächern 41 ist die Umfangsfläche wieder auf Radialnormalmaß, um Dichtzonen 43 auszubilden. Insgesamt verläuft die Dichtzone 43 vollständig um die Aufnahmevertiefung 41 herum.

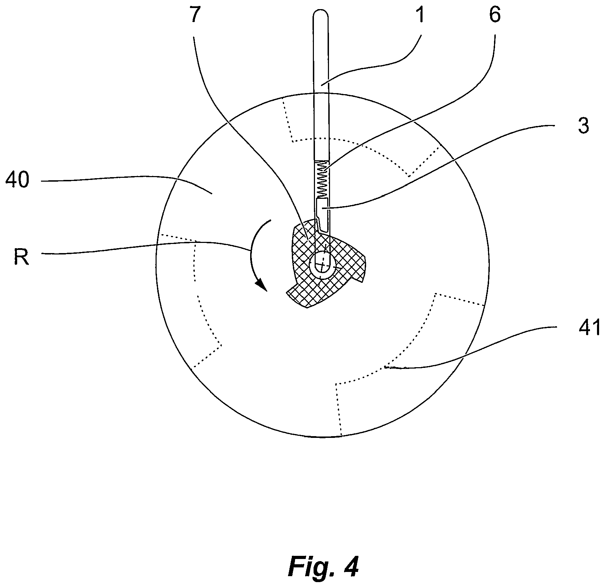

In Fig. 1 ist radial oberhalb der Rotationsachse R eine Verbindungs- und/oder Trenneinrichtung 48 als eine in der Trommelabrollfläche axial verlaufende Nut dargestellt. Von radial außen ist erfindungsgemäß ein nirgends dargestelltes Rakel und/oder Messer dazu bestimmt, in diese Nut einzufahren, sobald ein vollständiger Hub einer Transportvorrichtung 1 (Fig. 4, 5) erfolgt ist, der die Trommel 40 um eine Aufnahmevertiefung 41 weiter gedreht hat.

Die Umhüllungsmaterialien können erfindungsgemäß jeweils durch Falttechniken miteinander verbunden sein, dann führt der Messereingriff auch ohne echten Schnitt dazu, dass ein späterer Auswurf einer Einzelumhüllung gelingt. Es kann erfindungsgemäß alternativ im Fall echt kontinuierlich gefertigten Umhüllungsmaterials durch das Messer eine Perforierung eingebracht werden, bevorzugt bei gleichzeitiger Flächenpressung zur Verbesserung der Dichtwirkung.

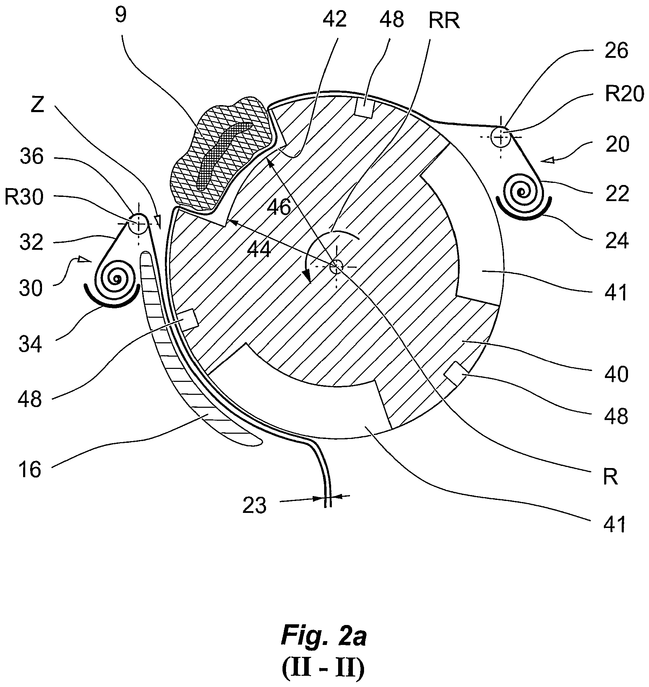

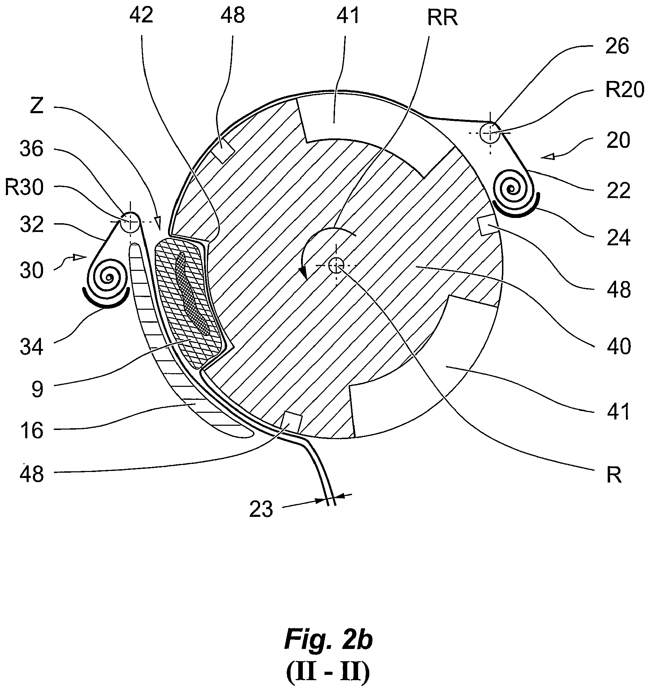

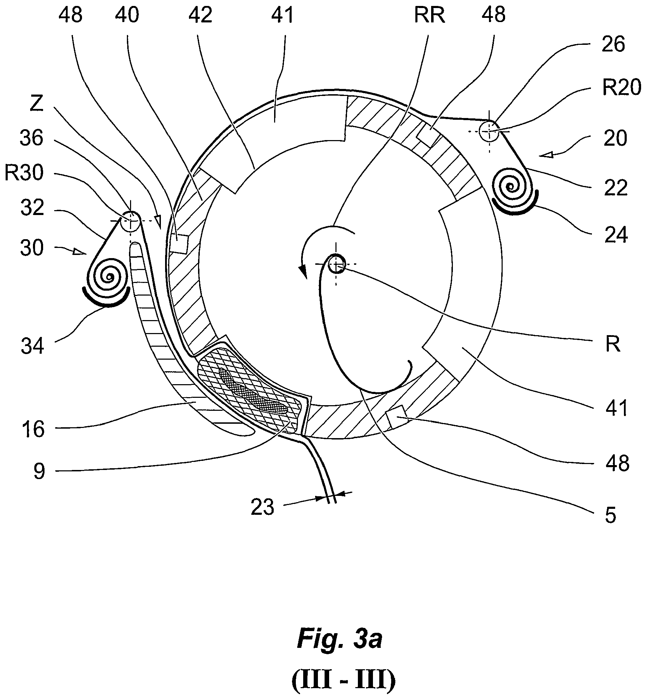

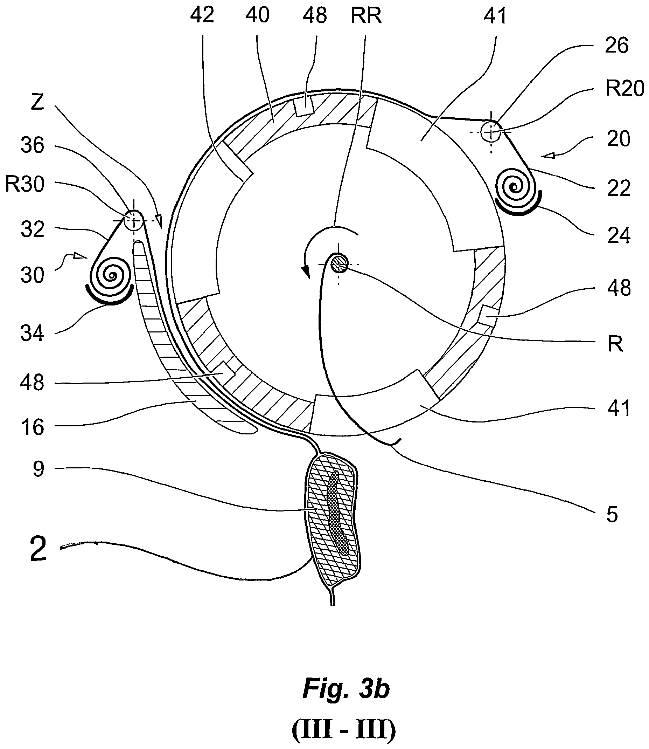

In den Figuren 2 und 3 umfassend die jeweiligen Darstellungen a und b ist ein Durchlauf eines Umhüllungsguts 9 in aufeinander folgenden Schritten gemäß Figurennummerierung 2a, 2b, 3a, 3b vom Befüllen 2a bis zum Ausferfen 3b gezeigt.

Diesen Darstellungen sind etwas detaillierter aber immer noch schematisch weitere funktionelle Teile der Magazine 20, 30 zu entnehmen. Je Magazin 20, 30 wird Umhüllungsmaterial 22, 32, beispielsweise als Materialrolle in einer Aufnahme 24, 34 gefasst. Erfindungsgemäße Aufnahmen fassen den Materialvorrat beispielsweise nur an Achsstummeln einer axial hinausstehenden Wickelachse. Dem abgewickelten Umhüllungsmaterial 22, 32 Richtung Trommel 40 folgend ist ein Materialtransport erneut mit Rollen 26, 36 angedeutet. Die Darstellung ist technisch jedoch nicht korrekt, weil ein Noppen-Näpfchen-Eingriff so nicht zu Stande käme. In einer erfindungsgemäßen und hier nicht dargestellten Ausführungsform wird sogar ganz auf solche Rollen 26, 28 verzichtet und alleine die Trommel 40 übernimmt die Transportfunktion für das Umhüllungsmaterial 22, 32.

Auch das Umhüllungsmaterial 22, 32 ist nur mit jeweils einer kräftigen Linie angedeutet. Es kann erfindungsgemäß jeweils mehrlagig ausgeführt sein. Ferner haben das erste Umhüllungsmaterial 22 und das zweite Umhüllungsmaterial 32 natürlich nach dem beschriebenen Kontakt in der Dichtzone 43 nicht mehr den gezeichneten Abstand 23 zueinander. Der eingezeichnete aber tatsächlich so nach dem Kontakt nicht mehr vorhandene Abstand 23 dient nur der Verdeutlichung des Materialflusses.

In den Zeichnungen der Figuren 2 und 3 ist ferner erkennbar, dass der Boden 42 einen kleinen Einlaufradius 44 und einen großen Auslaufradius 46 hat, um den Verdichtungsprozess zu unterstützen, der gemäß Fig. 2b zwischen Boden 42 und Gegenlage 16 stattfindet. Das Umhüllungsgut 9 läuft während des Verschließens der Umhüllungsmaterialien 22, 32 um das Umhüllungsgut 9 in einen Zwickel Z ein. In der Stellung gemäß Figur 3a ist der Verdichtungsprozess abgeschlossen und der Auswerfer 5 vorgespannt. In Fig. 3b tritt er seiner Entspannung folgend im zentralen Bereich 45 durch den Boden 42, um das Auswerfen zu unterstützen. Auch hier ist ein dazu erforderlicher Kontakt nicht dargestellt. Der Auswerfer 5 ist in Fig. 3b bereits vollständig entspannt und die Umhüllung hat die Aufnahmevertiefung 41 längst nach unten in den Behälter 10 verlassen. Beim Herunterfallen trennt sich die Umhüllung bevorzugt durch Abreißen an einer Perforation.

Oben wurde bereits das Fußpedal 18 und ein Hub der Transportvorrichtung 1 beschrieben. Ein Vorschlag zur erfindungsgemäßen Umsetzung einer solchen Ein-Hub-Mechanik ist mit Fig. 4 gemacht. Die Transportvorrichtung 1 umfasst einen Hebel. Der Hebel kann Teil eines Gestänges sein, das mit dem Fußpedal 18 zusammenwirkt. Alternativ umfasst die Erfindung einen Handhebel (nicht dargestellt).

Die Transportvorrichtung 1 umfasst eine Feder 6, die auf ein beweglich in der Transportvorrichtung 1 aufgenommenes Antriebselement 3 zu wirken bestimmt ist. Das Antriebselement 3 steht über eine Kraftübertragungseinrichtung 7 in mittelbarem Kontakt zur Trommel 40. Das Antriebselement 3 rastet gemäß der beispielhaft dargestellten Mechanik je Hub in der Kraftübertragungseinrichtung 7 ein.

### Bezugszeichenliste:

- 1: Transportvorrichtung
- 2: Umhüllung
- 3: Antriebselement
- 4: hinteres Ende
- 5: Auswerfer
- 7: Kraftübertragungseinrichtung
- 9: Umhüllungsgut

- 10: Behälter
- 12: Befüllöffnung
- 14: Aufsatz
- 16: Gegenlage
- 18: Fußpedal

- 20: erstes Magazin
- 22: erstes Umhüllungsmaterial
- 23: Abstand
- 24: erste Aufnahme Materialvorrat
- 26: erste Rolle
- 28: Napf

- 30: zweites Magazin
- 32: zweites Umhüllungsmaterial
- 34: zweite Aufnahme Materialvorrat
- 36: zweite Rolle
- 38: Napf

- 40: Trommel
- 41: Aufnahmevertiefung
- 42: Boden
- 43: Dichtzone
- 44: Einlaufradius
- 45: zentraler Bereich
- 46: Auslaufradius
- 47: Abtriebelement
- 48: Verbindungs- und/oder Trenneinrichtung
- 49: Noppe

- R: Rotationsachse (Trommel)
- R20: Umhüllungsrotationsachse
- R30: Umhüllungsrotationsachse
- RR: Rotationsrichtung
- Z: Zwickel

## Patentansprüche

1. Vorrichtung zum portionsweisen Verschließen von Exkrementen, Verbandsmaterialien, Damenhygieneartikeln oder dergleichen in jeweils einer Umhüllung (2) mit
- einem Behälter (10) oder einem Deckelaufsatz für einen Behälter mit zumindest einer Befüllöffnung (12) zum Aufnehmen zumindest eines zu verschließenden Objekts,
- einem zumindest teilweise in dem Behälter (10) oder dem Deckelaufsatz gelagerten Bewegungskoordinator für einen Vortrieb des Objekts in zumindest einer Bewegungsrichtung (O),
- zumindest einem Magazin (20, 30) zur Abgabe von Einhüllmaterial, wobei zumindest ein Oberflächenbereich des Bewegungskoordinators in Bewegungsrichtung (O) auf das Magazin (20, 30) folgend angeordnet ist,
- wobei entweder die Befüllöffnung (12) in Bewegungsrichtung (O) gesehen zwischen zumindest zwei Magazinen (20, 30) mit jeweils zumindest einem Einhüllmaterial angeordnet ist oder das zumindest eine Magazin (20, 30) zumindest zwei Einhüllmaterialien bevorratet, welche in Bewegungsrichtung (O) gesehen vor dem Erreichen der Befüllöffnung oder in der Befüllöffnung voneinander trennbar sind
- und mit einer Vereinigungseinrichtung, welche mit der Befüllöffnung zu kommunizieren bestimmt ist oder welche in Bewegungsrichtung (O) nach der Befüllöffnung (12) angeordnet ist, um die Einhüllmaterialien das Objekt umschließend miteinander zu verbinden,
**dadurch gekennzeichnet, dass** in einem zentralen Bereich (45) des Bewegungskoordinators zumindest ein Auswerfer (5) angeordnet ist und der Bewegungskoordinator in einem mittleren Bahnbereich zumindest bereichsweise offen ausgestaltet ist, sodass der Auswerfer (5), der dem zweiten Magazin (30) in Bewegungsrichtung (O) nachgeschaltet das umhüllte Material, dasselbe auswerfend erreicht, wobei bevorzugt das umhüllte Material den Auswerfer (5) gegen die Kraft einer Feder auszulenken bestimmt ist.

2. Vorrichtung nach Anspruch 1,
**dadurch gekennzeichnet, dass** der Bewegungsaktuator eine Trommel (40) ist, deren Rotationsachse (R) bevorzugt zumindest annähernd horizontal oder vertikal ausgerichtet ist.

3. Vorrichtung nach zumindest einem der Ansprüche 1 oder 2,
**dadurch gekennzeichnet, dass** die Magazine (20, 30) mit parallelen Einhüllmaterialrotationsachsen (R20, R30) angeordnet sind, bevorzugt für den Fall einer Ausbildung nach Anspruch 2 mit Einhüllmaterialrotationsachsen (R20, R30) parallel zu der Rotationsachse (R) der rotierbaren Trommel (40) ausgerichtet angeordnet sind.

4. Vorrichtung nach zumindest einem der Ansprüche 1 bis 3,
**dadurch gekennzeichnet, dass** zumindest ein Zwickel (Z) zwischen einer in dem Behälter (10) oder dem Deckelaufsatz befestigten oder ausgeformten Gegenlage (16) und einem Abschnitt eines die Befüllöffnung (12) umgebenden Rands ausgebildet ist.

5. Vorrichtung nach zumindest einem der Ansprüche 1 bis 4,
**dadurch gekennzeichnet, dass** der Befüllöffnung (12) zumindest ein in dem Bewegungskoordinator angeordneter Boden (42) zugeordnet ist, der bevorzugt je nach Stellung des Bewegungskoordinators flächenkongruent zur Befüllöffnung (12) angeordnet arretierbar (7, 41) ist.

6. Vorrichtung nach Anspruch 5,
**dadurch gekennzeichnet, dass** der Boden (42) in Bewegungsrichtung verjüngend angeordnet ist, insbesondere im Fall der Ausbildung des Bewegungskoordinators als Trommel (40) mit einem kleineren Einlaufradius und einen größeren Auslaufradius.

7. Vorrichtung nach einem der vorangehenden Ansprüche,
**dadurch gekennzeichnet, dass** das Umhüllungsmaterial vollständig gasdicht ist oder zumindest gasdicht ausgestattet ist.

8. Vorrichtung nach zumindest einem der Ansprüche 1 bis 6,
**dadurch gekennzeichnet, dass** der Bewegungskoordinator selbst über seine Oberfläche verteilt mehrere von Dichtzonen (43) umrandete Aufnahmevertiefungen (41) aufweist, wobei nach einer bevorzugten Ausführungsform die Dichtzonen (43) Teilflächen einer gedachten Umfangsfläche der Trommel (40) sind.

9. Vorrichtung nach den Ansprüchen 4 und 7,
**dadurch gekennzeichnet, dass** eine Andruckrolle des zweiten Magazins (30) Gegenlage für das Verdichten des Objekts (9) und Dichtandruckfläche zugleich ist.

10. Vorrichtung gemäß einem der vorangehenden Ansprüche,
**dadurch gekennzeichnet, dass** der Behälter (10) zumindest eines der folgenden Merkmale aufweist:
- gasdicht verschließbar,
- abschließbar, insbesondere mit einem Zahlenschloss,
- enthaltend einen Zähler und eine Schnittstelle zur Datenübertragung,
- aufweisend ein Display zur Anzeige der Füllmenge und / oder von Geruchsgefahr,
- aufweisend eine Kamera,
- aufweisend eine Fangschale für Feuchtigkeit, insbesondere mit einer Geruchssperrschicht, insbesondere Glycerin,
- doppelwandig mit Absorptionsmaterial zwischen den Wänden,
- aufweisend eine Beleuchtungseinrichtung und einen Energiespeicher, wobei der Energiespeicher beim Antreiben des Bewegungskoordinators und/oder durch Solarenergie aufladbar
ist,
- aufweisend eine Atmosphäre im Inneren, insbesondere bestehend aus sterilisierendem Gas,
- aufweisend eine Fallklappe mit Spannlippe als Dichtung, aufweisend ein Fenster mit Vergrößerungsglas zur intensiven Beleuchtung des Behältervolumens.

11. Umhüllungsverfahren zum portionsweisen Verschließen von Objekten (9), wie Exkremente, Verbandsmaterialien, Damenhygieneartikel oder dergleichen, in Umhüllungen (2), mittels einer Vorrichtung gemäß einem der Ansprüche 1-10, mit
- einer Transportvorrichtung (1) für ein erstes gasdichtes Umhüllungsmaterial (22), welche gemeinsam mit dem ersten Umhüllungsmaterial (22) eine Aufnahmevertiefung (41) auszubilden bestimmt ist,
- einem ein zweites gasdichtes Umhüllungsmaterial (32) aufweisenden Magazin, wobei bevorzugt ein einziges Magazin beide Umhüllungsmaterialien bevorratet,
- einer Verschlusseinrichtung, welche das Objekt (9) mittels zumindest Inkontaktbringen beider Umhüllungsmaterialien gasdicht zu verschließen bestimmt ist, und
- einem zum Verschließen bewegbaren Körper, wobei
- eine einzige Bewegung des Körpers erstens das erste Umhüllungsmaterial (22) aus einem ersten Magazin (20) entnimmt, zweitens das in die Aufnahmevertiefung (41) eingelegte Objekt (9) verdichtet und drittens zum Verschließen das zweite Umhüllungsmaterial (32) aus dem (20) oder einem zweiten Magazin (30) entnimmt und beide Umhüllungsmaterialien (22, 32) um die Aufnahmevertiefung (41) herum in einer Dichtzone (43) in Kontakt bringt, wobei der Antrieb des Körpers eine aktive Vakuumierungseinrichtung, insbesondere eine Pumpe, mitantreibt, und wobei ein Anschluss zwischen der Umhüllung (2) und der Vakuumierungseinrichtung solange geöffnet bleibt, bis ein hinteres Ende der Umhüllung (2) abgedichtet wird.

12. Umhüllungsverfahren nach Anspruch 12,
**dadurch gekennzeichnet, dass** um die Aufnahmevertiefung (41) vollständig herumgeführt in der Dichtzone (43) ein Kaltverschweißen stattfindet, um verdichtetes Umhüllungsgut (9) hermetisch gegenüber der Umwelt abzuriegeln.

13. Umhüllungsverfahren nach einem der Ansprüche 12 oder 13,
**dadurch gekennzeichnet, dass** das Verdichten dem Umhüllungsverfahren nachgeschaltet zunächst durch einen Schrumpfprozess verstärkt wird, wobei selbiges Schrumpfen ausgelöst wird durch einen Kontakt zwischen Umhüllungsmaterial (22, 32) und Umhüllungsgut (9), und dass sich eine Gasdichtheit der Umhüllungsmaterialien (22, 32) erst nach dem Schrumpfprozess einstellt.

14. Umhüllungsverfahren nach einem der vorangehenden Verfahrensansprüche, **dadurch gekennzeichnet, dass** je zu verschließender Portion Umhüllungsgut (9) ein einziger, antreibender Hub erforderlich ist, der auf die Transportvorrichtung (1) wirkt.

15. Vorrichtung oder Umhüllungsverfahren nach einem der vorangehenden Ansprüche,
**dadurch gekennzeichnet, dass** alle zum Umhüllen verwendeten Materialien (20, 30) biologisch abbaubar sind.

16. Verwendung der vorangehend beanspruchten Vorrichtungen oder Verfahren zum Portionieren und/oder Haltbarmachen von Energieträgern, insbesondere zum gasdichten Portionieren.

## Claims

1. Device for the enclosing of portions of excrement, bandaging materials, feminine hygiene articles or the like, each in a wrapping (2), with
- a container (10) or a lid for a container with at least one fill opening (12) to receive at least one object being sealed,
- a movement coordinator at least partly mounted in the container (10) or the lid for a propelling of the object in at least one direction of movement (O),
- at least one magazine (20, 30) for dispensing of wrapping material, wherein at least one surface region of the movement coordinator is arranged in the direction of movement (O) toward the magazine (20, 30),
- wherein either the fill opening (12) looking in the direction of movement (O) is arranged between at least two magazines (20, 30) each with at least one wrapping material or the at least one magazine (20, 30) stockpiles at least two wrapping materials, which can be separated from each other before reaching the fill opening or in the fill opening, looking in the direction of movement (O)
- and with a joining device, which is designed to communicate with the fill opening or which is arranged after the fill opening (12) in the direction of movement (O), in order to join together the wrapping materials enclosing the object,
**characterized in that** in a central region (45) of the movement coordinator at least one ejector (5) is arranged and the movement coordinator is at least partly open in a middle travel region, so that the ejector (5) coming after the second magazine (30) in the direction of movement (O) reaches the wrapped material and ejects it, and preferably the wrapped material is designed to deflect the ejector (5) against the force of a spring.

2. Device according to claim 1,
**characterized in that** the movement actuator is a drum (40), whose axis of rotation (R) is preferably oriented at least approximately horizontally or vertically.

3. Device according to at least one of claims 1 or 2,
**characterized in that** the magazines (20, 30) are arranged with parallel wrapping material axis of rotation (R20, R30), preferably for the case of an embodiment according to claim 2 with wrapping material axis of rotation (R20, R30) parallel to the axis of rotation (R) of the rotatable drum (40).

4. Device according to at least one of claims 1 to 3,
**characterized in that** at least one gusset (Z) is formed between a bearing surface (16) secured or formed in the container (10) and the lid and a segment of a margin enclosing the fill opening (12).

5. Device according to at least one of claims 1 to 4,
**characterized in that** the fill opening (12) is coordinated with at least one bottom (42) arranged in the movement coordinator, which can be locked (7, 41) by being arranged roughly congruent to the surface of the fill opening (12), depending on the position of the movement coordinator.

6. Device according to claim 5,
**characterized in that** the bottom (42) when viewing a cross section along the direction of movement (O) is arranged to narrow the fill opening (12) in the direction of movement, especially in the case when the movement coordinator is configured as a drum (40) with a smaller inlet radius (44) and a larger outlet radius (46).

7. Device according to one of the preceding claims,
**characterized in that** the wrapping material is completely gas-tight or at least outfitted to be gas-tight.

8. Device according to at least one of claims 1 to 6,
**characterized in that** the movement coordinator itself has receiving recesses (41) bordered by a plurality of sealing zones (43) distributed about its surface, while according to a preferred embodiment the sealing zones (43) are partial surfaces of an imaginary circumferential surface of the drum (40).

9. Device according to claims 4 and 7,
**characterized in that** a pressure roll of the second magazine (30) is a bearing surface for the sealing of the object (9) and a sealing pressure surface at the same time.

10. Device to at least one of the preceding claims,
**characterized in that** the container (10) has at least one of the following features:
- can be sealed gas-tight,
- can be closed, in particular, with a combination lock,
- containing a counter and an interface for data transmission,
- having a display to show the full amount and/or danger of odor,
- having a camera,
- having a trap bowl for moisture, especially with an odor blocking layer, especially glycerin,
- double-walled with absorption material between the walls,
- having a lighting fixture and an energy storage, the energy storage being chargeable during the driving of the drum and/or by solar energy,
- having an atmosphere on the inside, especially a sterilizing gas,
- having a trap door with clamping lip as a seal, having a window with magnifying glass for intensive illumination of the container volume.

11. Wrapping method for the enclosing of portions of objects (9) such as excrement, bandaging materials, feminine hygiene products or the like, in wrappings (2), with
- a transport device (1) for a first gas-tight wrapping material (22), which together with the first wrapping material (22) is designed to form a receiving recess (41),
- a magazine having a second gas-tight wrapping material (32), wherein preferably a single magazine stockpiles both wrapping materials,
- a sealing mechanism, which is designed to seal the object (9) gas-tight by at least bringing both wrapping materials into contact, and
- a movable body for the closing process, wherein
a single movement of the body first of all removes the first wrapping material (22) from a first magazine (20), secondly seals the object (9) placed in the receiving recess (41) and thirdly removes the second wrapping material (32) from the [first magazine] (20) or a second magazine (30) for the sealing and brings both wrapping materials (22, 32) into contact in a sealing zone (43) around the receiving recess (41), **characterized in that** the driving of the body entrains an active evacuating mechanism, especially a pump, and a connection between the wrapping (2) and the evacuating mechanism remains open until a rear end (4) of the wrapping (2) is sealed.

12. Wrapping method according to claim 12,
**characterized in that** a cold welding takes place completely around the receiving recess (41) in the sealing zone (43) in order to block off the sealed wrapping item (9) hermetically from the surroundings.

13. Wrapping method according to one of claims 12 or 13,
**characterized in that** the sealing coming after the wrapping process is at first intensified by a shrinkage process, wherein said shrinkage is triggered by a contact between wrapping material (22, 32) and wrapping item (9), and a gas tightness of the wrapping materials (22, 32) occurs only after the shrinkage process.

14. Wrapping method according to one of the preceding method claims, **characterized in that** a single driving stroke is required for each portion of wrapping item (9) being closed, acting upon the transport device (1).

15. Device or wrapping method according to one of the preceding claims,
**characterized in that** all materials (20, 30) used for the wrapping are biodegradable.

16. Use of the above claimed devices or methods for portioning and/or preserving of energy-rich materials, especially for gas-tight portioning.

## Revendications

1. Dispositif pour l'enfermement par portions d'excréments, de matériaux de bandage, d'articles hygiéniques pour femmes ou articles similaires dans respectivement une enveloppe (2) avec
- un récipient (10) ou un élément de couvercle de récipient avec au moins une ouverture de remplissage (12) pour la réception d'au moins un objet à enfermer,
- un coordinateur de mouvement se trouvant au moins en partie dans le récipient (10) ou dans l'applique de couvercle pour l'entraînement dans l'objet dans au moins un sens de mouvement (O),
- au moins au magasin (20, 30) pour la délivrance du matériau d'enveloppement, dans lequel cas au moins une zone de la surface du coordinateur de mouvement est arrangée en succession au sens de mouvement (O) du magasin (20, 30),
- dans lequel cas soit l'ouverture de remplissage (12) vue dans le sens du mouvement (O) est arrangée entre deux magasins (20, 30) avec chacun au moins un matériau d'enveloppement ou soit au moins un magasine (20, 30) approvisionne au moins deux matériaux d'enveloppement, lesquels vus dans le sens du mouvement (O) sont séparés les uns des autres avant d'atteindre l'ouverture de remplissage ou dans l'ouverture de remplissage
- et avec un dispositif de jonction, lequel est destiné à communiquer avec l'ouverture de remplissage, lequel est arrangé dans le sens du mouvement (O) après l'ouverture de remplissage (12) afin de fusionner pour finir les matériaux d'enveloppement autour de l'objet,
**caractérisé en ce que** dans une zone centrale (45) du coordinateur de mouvement au moins un dispositif d'éjection (5) placé et le coordinateur de mouvements soient au moins conçus en partie de manière ouverte sur une zone de bande centrale de telle sorte que le dispositif d'éjection (5) placé après le deuxième magasin (30) dans le sens du mouvement (O) accède au matériau développé en le rejetant, dans lequel cas le matériau enveloppé est à sortir de manière préférentielle du dispositif d'éjection (5) contre la force d'un ressort.

2. Dispositif selon la revendication 1,
**caractérisé en ce que** l'actionneur de mouvement est un tambour (40), lequel axe de rotation (R) est aligné de manière préférentielle au mois approximativement à l'horizontale ou à la verticale

3. Dispositif selon au moins une des revendications 1 ou 2,
**caractérisé en ce que** le magasin (20, 30) est arrangé avec des axes parallèles de rotation du matériau d'enveloppement (R20, R30), de manière alignée en préférence pour le cas d'une formation selon la revendication 2 avec des axes de rotation du matériau d'enveloppement (R20, R30) en parallèle aux axes de rotation (R) des tambours rotatifs (40).

4. Dispositif selon au moins une des revendications 1 à 3,
**caractérisé en ce que** au moins un gousset (Z) soit formé entre un contre-appui (16) dans le récipient (10) ou fixé ou formé à l'applique de couvercle et une section de la bordure entourant l'ouverture de remplissage (12).

5. Dispositif selon au moins une des revendications 1 à 4,
**caractérisé en ce que** l'ouverture de remplissage (12) soit affectée à au moins un fond (42) placé dans le coordinateur de mouvements, lequel est arrangé de manière pouvant être verrouillée (7,41) à l'ouverture de remplissage (12) de manière à peu près appropriée à la surface selon la position du coordinateur de mouvement.

6. Dispositif selon la revendication 5,
**caractérisé en ce que** le fond (42) est arrangé avec une ouverture de remplissage (12) se rétrécissant en considération d'une découpe le long du sens de mouvement (O), en particulier dans le cas de la formation du coordinateur de mouvement comme tambour avec un rayon plus petit d'entrée (44) et un rayon plus grand d'évacuation (46).

7. Dispositif selon une des précédentes revendications,
**caractérisé en ce que** le matériau d'enveloppement est complètement étanche aux gaz ou au moins équipé de manière étanche aux gaz.

8. Dispositif selon au moins une des revendications 1 à 6,
**caractérisé en ce que** le coordinateur de mouvements présente lui-même de manière répartie sur sa surface plusieurs cavités d'accueil (41) entourées de zones d'étanchéité (43) dans lequel cas sous une forme privilégiée de réalisation les zones d'étanchéité (43) sont des surfaces partielles d'une périphérie réfléchie du tambour (40)

9. Dispositif selon les revendications 4 et 7,
**caractérisé en ce que** un rouleau de pression du deuxième magasin (30) est simultanément un contre-appui pour le compactage de l'objet (9) et une surface d'appui pour l'étanchéité.

10. Dispositif selon une des revendications ci-dessus, **caractérisé en ce que** le récipient (10) présente au moins une des caractéristiques suivantes :
- refermable de manière étanche aux gaz,
- verrouillable, en particulier par une serrure à combinaison,
- comprenant un compteur et une interface pour la transmission des données,
- présentant un écran pour l'affichage de la quantité remplie et/ou de risques d'odeur,
- présentant une caméra,
- présentant une coupelle de réception de l'humidité, en particulier avec un revêtement anti-odeurs, en particulier de la glycérine,
- à double paroi avec matériau d'absorption entre les parois,
- présentant un dispositif d'éclairage et un accumulateur d'énergie qui peut être rechargé lors de l'entraînement du coordinateur de mouvements et/ou de l'énergie photovoltaïque,
- présentant à l'intérieur une atmosphère, en particulier un gaz de stérilisation,
- présentant un clapet de chute avec des lèvres de serrage comme joint, présentant une fenêtre avec une loupe grossissante pour un éclairage intensif du volume du récipient

11. Dispositif pour l'enfermement à portions d'objets (9), tels que les excréments, les matériaux de bandage, les articles hygiéniques pour femmes et articles similaires dans des emballages (2) avec
- Un dispositif de transport (1) pour un premier matériau d'enveloppement imperméable au gaz (22), lequel est destiné à former en commun avec le premier matériau d'enveloppement (22) une cavité d'accueil (41),
- Un deuxième magasin présentant un deuxième matériau d'enveloppement imperméable aux gaz (32), dans lequel cas de manière préférentielle un seul magasin approvisionne les deux matériaux d'enveloppement,
- Un dispositif de mise sous scellé qui est destiné à refermer l'objet (9) au moins en mettant en contact de manière imperméable aux gaz les deux matériaux d'enveloppement, et
- Un corps mobile pour la fermeture, dans lequel cas un seul et unique mouvement du corps prélève premièrement le premier matériau d'enveloppement (22) du premier magasin (20), compacte deuxièmement l'objet (9) déposé dans la cavité d'accueil (41) et troisièmement prélève pour la fermeture le deuxième matériau d'enveloppement (32) du magasin (20) ou d'un deuxième magasin (30) et met en contact les deux matériaux d'enveloppement (22, 32) dans une zone d'étanchéité (43) autour de la cavité d'accueil (41), **caractérisé en ce que** l'entraînement du corps entraine conjointement un dispositif actif de mise sous vide, en particulier une pompe et que le raccordement entre la mise sous enveloppe (2) et le dispositif de mise sous vide reste ouvert, jusqu'à ce qu'une extrémité arrière (4) de l'enveloppe (2) soit scellée de manière étanche.

12. Procédure d'enveloppement selon la revendication 12,
**caractérisée en ce qu'**un soudage à froid a lieu dans la zone d'étanchéité (43) laquelle est conduite entièrement autour de la cavité d'accueil (41) afin de sceller de manière hermétique à l'environnement un bien enrobé compacté (9).

13. Procédure d'enveloppement selon une des revendications 12 ou 13,
**caractérisée en ce que** le compactage faisant suite à la procédure d'enveloppement est tout d'abord renforcé par un processus de rétrécissement, dans lequel cas le même rétrécissement est déclenché par un contact entre le matériau d'enveloppement (22, 32) et le bien enveloppé (9), de telle sorte qu'une imperméabilité aux gaz (22, 32) n'intervient que seulement après le processus de rétrécissement.

14. Procédure d'enveloppement selon une des revendications ci-dessus de la procédure, **caractérisée en ce qu'**une seule pression d'entraînement est nécessaire pour chaque portion de bien enveloppé à refermer (9), laquelle pression fait effet sur le dispositif de transport (1).

15. Dispositif ou procédure d'enveloppement selon une des revendications ci-dessus,
**caractérisée en ce que** tous les matériaux (20, 30) utilisés pour d'enveloppement sont biodégradables

16. Utilisation de la procédure ou du dispositif revendiqué ci-dessus pour la mise en portions et/ou la conservation de supports énergétique, en particulier une mise en portions imperméables aux gaz
